# EUROPEAN PATENT SPECIFICATION

(11) **EP 2 412 797 B1**
(45) Date of publication and mention of the grant of the patent: **17.01.2018**
(21) Application number: 10755846.2
(22) Date of filing: 09.03.2010
(51) Int. Cl.: C12N 1/20, A61K 8/99, A61K 35/74, A61P 37/04, A61P 43/00, C12R 1/225

(54) **METHOD FOR PRODUCING LACTIC ACID BACTERIA HAVING ENHANCED IMMUNOMODULATING ACTIVITIES**
VERFAHREN ZUR HERSTELLUNG VON MILCHSÄUREBAKTERIEN MIT VERSTÄRKTEN IMMUNOMODULIERENDEN WIRKUNGEN
MÉTHODE DE PRODUCTION DE BACTÉRIES LACTIQUES PRÉSENTANT DES ACTIVITÉS IMMUNOMODULATRICES AMÉLIORÉES

(30) Priority: 24.03.2009 JP 2009071213; 15.05.2009 JP 2009119295
(43) Date of publication of application: 01.02.2012
(73) Proprietor: Suntory Holdings Limited, Kita-ku, Osaka-shi Osaka 530-8203 (JP)
(72) Inventor: IZUMO, Takayuki, Mishima-gun Osaka 618-8503 (JP); HOSHIKO, Hiroyuki, Mishima-gun Osaka 618-8503 (JP); MAEKAWA, Toshihiro, Mishima-gun Osaka 618-8503 (JP)
(74) Representative: Vossius & Partner Patentanwälte Rechtsanwälte mbB
(86) International application number: PCT/JP2010/053846
(87) International publication number: WO 2010/110045

(56) References cited:
- WO-A1-2008/023665
- JP-A- 2006 028 047
- JP-A- 2008 179 630
- JP-A- 2008 245 576
- K ADAMBERG: "The effect of temperature and pH on the growth of lactic acid bacteria: a pH-auxostat study", INTERNATIONAL JOURNAL OF FOOD MICROBIOLOGY, vol. 85, no. 1-2, 15 August 2003 (2003-08-15), pages 171-183, XP055112669, ISSN: 0168-1605, DOI: 10.1016/S0168-1605(02)00537-8
- KOTANI S ET AL: "Immunoadjuvant activities of peptidoglycan subunits from the cell walls of Staphyloccus aureus and Lactobacillus plantarum.", BIKEN JOURNAL, vol. 18, no. 2, 1 June 1975 (1975-06-01), pages 93-103, XP009177488, ISSN: 0006-2324
- GLAASKER E ET AL: "Physiological response of Lactobacillusplantarum to salt and nonelectrolyte stress", JOURNAL OF BACTERIOLOGY, AMERICAN SOCIETY FOR MICROBIOLOGY, WASHINGTON, DC; US, vol. 180, no. 17, 1 January 1998 (1998-01-01), pages 4718-4723, XP009135496, ISSN: 0021-9193
- SIMOVA E ET AL: "Amino acid profiles of lactic acid bacteria, isolated from kefir grains and kefir starter made from them", INTERNATIONAL JOURNAL OF FOOD MICROBIOLOGY, ELSEVIER BV, NL, vol. 107, no. 2, 15 March 2006 (2006-03-15), pages 112-123, XP024956475, ISSN: 0168-1605, DOI: 10.1016/J.IJFOODMICRO.2005.08.020 [retrieved on 2006-03-15]
- MATTARELLI, P. ET AL.: 'Effect of growth temperature on the biosynthesis of cell wall proteins from Bifidobacterium globosum' RES. MICROBIOL. vol. 150, 1999, pages 117 - 127, XP008153197
- MASAKO OSUMI: 'Shinkin Saiboheki no Keisei Katei' THE CELL vol. 33, no. 14, 2001, pages 550 - 552, XP008153222
- HIROMI KIMOTO ET AL.: 'Men'eki Fukatsu Sayo o Yusuru Lactococcus Nyusankin no Kintai Seibun no Kaiseki' MILK SCIENCE vol. 299, no. 55, 2006, page 102, XP001526054
- VICTORIA M. ET AL.: 'Proinflammatory cytokine and nitric oxide induction in murine macrophages by cell wall and cytoplasmic extracts of lactic acid bacteria' J.FOOD PROT. vol. 62, no. 12, 1999, pages 1435 - 1444, XP008153191
- AMBROSINI, V.M. ET AL.: 'Immunostimulating activity of cell walls from lactic acid bacteria and related species' FOOD AGRI. IMMUNOL. vol. 10, 1998, pages 183 - 191, XP008153192
- ZWIETERING M H ET AL: "Modeling of the bacterial growth curve", APPLIED AND ENVIRONMENTAL MICROBIOLOGY, AMERICAN SOCIETY FOR MICROBIOLOGY, US, vol. 56, no. 6, 1 June 1990 (1990-06-01), pages 1875-1881, XP002664178, ISSN: 0099-2240
- NONAKA YUJI ET AL: "Antiallergic effects of Lactobacillus pentosus strain S-PT84 mediated by modulation of Th1/Th2 immunobalance and induction of IL-10 production", INTERNATIONAL ARCHIVES OF ALLERGY AND IMMUNOLOGY,, vol. 145, no. 3, 1 January 2008 (2008-01-01), pages 249-257, XP009129049, ISSN: 1423-0097

### SEQUENCE LISTING

<110> Suntory Holdings Ltd.
<120> Method of producing lactic acid bacteria having enhanced immunoregulatory function
<130> FA0008-10004
<150> JP 2009-071213
   <151> 20090324
<150> JP 2009-119295
   <151> 20090515
<160> 8
<170> PatentIn version 3.1
<210> 1
   <211> 19
   <212> DNA
   <213> primer
<400> 1
   aggaaggtcc taagtggca 19
<210> 2
   <211> 19
   <212> DNA
   <213> primer
<400> 2
   actcgctcca acccttcat 19
<210> 3
   <211> 20
   <212> DNA
   <213> primer
<400> 3
   gccgtcgtct caatcgaaga 20
<210> 4
   <211> 20
   <212> DNA
   <213> primer
<400> 4
   gtaccagtct taccagcttg 20
<210> 5
   <211> 20
   <212> DNA
   <213> primer
<400> 5
   gcgatgtatt tgaataacgc 20
<210> 6
   <211> 20
   <212> DNA
   <213> primer
<400> 6
   agcatcatac tggtcatttc 20
<210> 7
   <211> 20
   <212> DNA
   <213> primer
<400> 7
   accgacttcg ggtgttacaa 20
<210> 8
   <211> 20
   <212> DNA
   <213> primer
<400> 8
   cgcctacatg aagtcggaat 20

## Description

### TECHNICAL FIELD

The present invention relates to a method for producing lactic acid bacteria having enhanced immunoregulating activities. More specifically, the present invention relates to a method for producing lactic acid bacteria having immunoregulating activities, such as an immunostimulating activity and an antiallergy activity, which are enhanced by increasing the cell walls of the lactic acid bacteria, that is, the immunoregulating components,.

### BACKGROUND ART

Cases of infectious diseases caused by immunological deterioration due to aging are increasing recently. Immunological deterioration is also caused by living in the busy modern society and in a stressful environment. A measure against such deterioration is strongly desired. Another recent problem is the spread of emerging infectious diseases, such as avian flu, and reemerging infection diseases, such as tuberculosis. Consequently, the enhancement of immunity, which protects human bodies from foreign enemies, is essential for the modern people living with risks of their immunological competency deteriorating. This situation urgently calls for the development of safe food products that exhibit immunoregulating activities over a long time period and that enable people to maintain their health in stressful environments.

A variety of lactic acid bacteria and dairy products containing these bacteria are commercialized as food materials possessing immunoregulating activities. These lactic acid bacteria are bacteria of *Lactobacillus, Lactococcus, Streptococcus, Pediococcus, Enterococcus* or other genera, and their possession of an immunostimulating activity or an antiallergy activity are known in the art (Patent Documents 1 to 4). However, the immunostimulating activities disclosed in the above patent documents are results of in vitro tests or animal tests, and no findings concerning actual immunoregulating activities in a human are disclosed.

Further, methods for enhancing the immunoregulating activities of lactic acid bacteria include a method using a culture medium of corn steep liquor for cultivation (Patent Document 5), a method using a culture medium containing salt at a ratio of 5 to 15% (Patent Document 6), a method using a culture medium containing a surfactant at a ratio of 0.1 to 1% and carbonate at a ratio of 0.01% to 0.1% wherein the heating is carried out at 80 to 120°C for 5 to 30 minutes (Patent Document 7). However, an increase in materials as well as the number of steps makes these methods costly.

Moreover, Non-Patent Document (Nonaka, et al.; Int. Allergy Immunol. 2008; 145: 249-257) reports on antiallergic effects of Lactobacillus pentosus strain S-PT84 mediated by Modulation of Th1/Th2 immunobalance and induction of IL-10 production.

### CITATION LIST

### PATENT DOCUMENT

Patent Document 1: Japanese Patent Public Disclosure (Kokai) No. 2004-18469
Patent Document 2: Japanese Patent Public Disclosure (Kokai) No. H06-80575
Patent Document 3: Japanese Patent No. 3174611
Patent Document 4: Japanese Patent Public Disclosure (Kokai) No. 2000-95697
Patent Document 5: Japanese Patent No. 4115181
Patent Document 6: Japanese Patent Public Disclosure (Kokai) No. 2006-28047
Patent Document 7: Japanese Patent Public Disclosure (Kokai) No. 2007-131610

Non-Patent Document 1: Nonaka, et al.

### SUMMARY OF INVENTION

### TECHNICAL PROBLEM

As mentioned above, there is an urgent call for the development of safe food products that exhibit immunoregulating activities over a long time period and that enable people to maintain their health in stressful environments.

The present invention, in view of the above problem, aims to provide a method for producing lactic acid bacteria that exhibit excellent immunoregulating activities in a human. Products comprising such bacteria including foods and drinks, medicines, and cosmetics by enhancing immunoregulating functions of lactic acid bacteria without requiring any special medium or any any special process are disclosed herein.

### SOLUTION TO PROBLEM

The present inventors found that cultivations of lactic acid bacteria at different temperatures change the growth of the lactic acid bacteria, and that changes in the cell wall synthetic enzyme genes, the amount of cell wall components, and the thickness of cell walls are caused in association with the change in growth of the bacteria.

A further study gave a surprising result that the content and the thickness of cell walls increase when lactic acid bacteria are cultivated at a temperature higher than the recommended cultivation temperature of the bacteria to be used, and the immunoregulating activities improve in correlation to these increases. The present invention was accomplished on the basis of these findings.

The present invention is summarized by items 1 to 3 below.
1. A method for producing lactic acid bacteria having enhanced immunoregulating activities comprising the step of:
   cultivating lactic acid bacteria under an osmotic pressure of 500 to 1000 mOsm and at a cultivation temperature that is at least 1°C higher than a recommended cultivation temperature of the lactic acid bacteria and that gives a bacterial concentration 25 to 95% of a bacterial concentration obtained by cultivation at the recommended cultivation temperature, thereby lactic acid bacteria having enhanced immunoregulating activities are obtained,
   wherein the lactic acid bacteria are bacteria of *Lactobacillus pentosus, Lactobacillus brevis, Lactobucillus fermentum* or *Lactobacillus casei*,
   wherein for *Lactobacillus pentosus* or *Lactobacillus brevis* the recommended cultivation temperature is 30°C and the cultivation temperature is 31 °C to 41 °C,
   and wherein for *Lactobacillus fermentum* or *Lactobacillus casei* the recommended cultivation temperature is 37°C and the cultivation temperature is 41 °C to 44°C.
2. A method for producing lactic acid bacteria having enhanced immunoregulating activities comprising the step of:
   cultivating lactic acid bacteria under an osmotic pressure of 500 to 1000 mOsm and at a cultivation temperature that increases diaminopimelate in a cell wall to at least 1.35 times in comparison to an amount obtained by cultivation of the lactic acid bacteria at a recommended cultivation temperature, or at a cultivation temperature that increases a cell wall thickness to at least 106% in comparison to a cell wall thickness obtained by cultivation of the lactic acid bacteria at a recommended cultivation temperature, thereby lactic acid bacteria having enhanced immunoregulating activities are obtained,
   wherein the lactic acid bacteria are bacteria of *Lactobacillus pentosus, Lactobacillus brevis, Lactobacillus fermentum* or *Lactobacillus casei*,
   wherein for *Lactobacillus pentosus* or *Lactobacillus brevis* the recommended cultivation temperature is 30°C and the cultivation temperature is 31 °C to 41 °C,
   and wherein for *Lactobacillus fermentum* or *Lactobacillus casei* the recommended cultivation temperature is 37°C and the cultivation temperature is 41 °C to 44°C.
3. The method according to item 1 or 2, wherein the lactic acid bacteria is *Lactobacillus pentosus* S-PT84 or *Lactobacillus pentosus* JCM1558^{T}.

### ADVANTAGEOUS EFFECTS OF THE INVENTION

Lactic acid bacteria produced by the method of the present invention or compositions comprising the same
are produced from food materials, so they are extremely safe and they can be consumed daily or continuously with an interval of an appropriate number of days, over a short time period or a long time period. Thus, the bacteria consumed as foods or drinks or as health foods can prevent the deterioration of immunological functions due to various causes by regulating immunological functions for a long time period. Further, such bacteria can prevent excessive stimulation of immunological functions that have negative effects on the living body by regulating the balance of immunological functions.

The lactic acid bacteria produced by the method of the present invention can be used as medicines, and they
exert mild effects that can reduce or cure various symptoms caused by the deterioration or excessive stimulation of immunological functions.

### BRIEF DESCRIPTION OF THE DRAWINGS

[Figure 1] Figure 1 shows the bacterial concentration of *Lactobacillus pentosus* S-PT84 (FERM BP-10028) after cultivation has ended.
[Figure 2] Figure 2 shows the bacterial concentration of *Lactobacillus pentosus* JCM1558^{T} after cultivation has ended.
[Figure 3] Figure 3 shows the measured result of diaminopimelate (DAP), which is amino acid that constitutes the cell wall.
[Figure 4] Figure 4 shows variations in IL-12 inducibility due to differences in the cultivation temperature (*Lactobacillus pentosus* S-PT84).
[Figure 5] Figure 5 shows variations in IL-12 inducibility due to differences in the cultivation temperature (*Lactobacillus pentosus* JCM1558^{T}).
[Figure 6] Figure 6 shows the bacterial concentration of *Lactobacillus plantarum* JCM1149^{T} after cultivation has ended (Reference Example).
[Figure 7] Figure 7 shows variations in IL-12 inducibility due to differences in the cultivation temperature (*Lactobacillus plantarum* JCM1149^{T}) (Reference Example).
[Figure 8] Figure 8 shows the bacterial concentration of *Lactobacillus casei* JCM1134^{T} after cultivation has ended.
[Figure 9] Figure 9 shows variations in IL-12 inducibility due to differences in the cultivation temperature (*Lactobacillus casei* JCM1134^{T}).
[Figure 10] Figure 10 shows variations in the expression level of cell wall synthetic enzyme genes due to differences in the cultivation temperature.
[Figure 11] Figure 11 shows variations in the expression level of cell wall synthetic enzyme genes due to differences in osmotic pressure (30°C).
[Figure 12] Figure 12 shows variations in the expression level of cell wall synthetic enzyme genes due to differences in osmotic pressure (37°C).
[Figure 13] Figure 13 shows the measured result of the cell wall thickness.
[Figure 14] Figure 14 shows the measured result of IL-12 inducibility of lactic acid bacteria having cell walls of different thicknesses (using bacterial cells of Figure 13).
[Figure 15] Figure 15 shows the amount of cell wall components and the IL-12 inducibility of the lactic acid bacteria cultivated at an actual production scale (1000 L).
[Figure 16] Figure 16 shows the test result of immunostimulating effects in a human (the change in the NK activity when the NK activity before intake of the bacteria is 100).
[Figure 17] Figure 17 shows the test result of immunostimulating effects in a human (the change in the amount of IFN-α production from before intake of the bacteria).
[Figure 18] Figure 18 shows the test result of immunostimulating activity in a human (the change in the Th1/Th2 ratio from before intake of the bacteria).
[Figure 19] Figure 19 shows the cell wall thickness and the IL-12 inducibility of various lactic acid bacteria.

### DESCRIPTION OF EMBODIMENTS

The embodiments of the present invention are described in detail below.

### <Method for Producing Lactic Acid Bacteria Having Enhanced Immnoregulating Activities>

### Lactic Acid Bacteria

The Lactic acid bacteria *Lactobacillus pentosus*, *Lactobacillus brevis, Lactobacillus fermentum or Lactobacillus casei* are used in the present invention.

Multiple types of bacteria may be used in combination as lactic acid bacteria.

### Cultivation Temperature

The method of the present invention comprises a step of cultivating lactic acid bacteria at a temperature that is at least 1°C higher than the recommended cultivation temperature and that gives a bacterial concentration 25 to 95% of the concentration obtained by cultivation at the recommended cultivation temperature. The method comprises a step of cultivating lactic acid bacteria preferably at a temperature that gives a bacterial concentration 30 to 95% of the concentration obtained by cultivation at the recommended cultivation temperature, and more preferably at temperatures that give bacterial concentrations 35 to 95%, 40 to 95%, 45 to 95%, 50 to 95% of the concentration obtained by cultivation at the recommended cultivation temperature. Cultivation under such conditions enables production of lactic acid bacteria having enhanced immnoregulating activities.

The recommended cultivation temperature is the cultivation temperature recommended for each lactic acid bacterium. Lactic acid bacteria may be classified into plant derived lactic acid bacteria and animal derived lactic acid bacteria. Generally speaking, the recommended cultivation temperature of the plant derived lactic acid bacteria is 30°C, and the recommended cultivation temperature of the animal derived lactic acid bacteria is 37°C. Specifically, the recommended cultivation temperature of *Lactobacillus pentosus* S-PT84 (FERM BP-10028) and *Lactobacillus pentosus* JCM1558^{T}, which are plant derived lactic acid bacteria, is 30°C.

"A temperature that provides a bacterial concentration 25 to 95% of the concentration obtained by cultivation at the recommended cultivation temperature" is a cultivation temperature that gives a maximum attainable bacterial cell concentration that is 25 to 95% in comparison to the maximum attainable bacterial cell concentration of cultivation at a recommended cultivation temperature (specifically, 30°C or 37°C). The time required to reach the maximum attainable bacterial cell concentration is generally about 18 to 36 hours, so the above temperature can be determined by comparing, for example, the bacterial concentrations of after 24 hours of cultivation.

The preferable cultivation temperature differs for each bacterial strain. In view of immunostimulating activities and bacterial concentrations to be obtained, the cultivation temperature for bacteria having a recommended cultivation temperature of 30°C is 31 to 41°C, which ensures the production of a sufficient number of bacteria and enables bacterial cells to be prepared with high immunostimulating activities, and a more preferable temperature range is 32°C to 40°C. In view of immunostimulating activities and bacterial concentrations to be obtained, the cultivation temperature for bacteria having a recommended cultivation temperature of 37°C is 41 to 44°C, which ensures the production of a sufficient number of bacteria and enables bacterial cells with high immunostimulating activities to be prepared.

"A cultivation temperature that increases the amount of diaminopimelate in the cell wall to at least 1.35 times in comparison to the amount obtained by cultivation at the recommended cultivation temperature or that increases the cell wall thickness to at least 106% in comparison to the thickness obtained by cultivation at the recommended cultivation temperature" is a temperature that makes the amount of diaminopimelate at least 1.35 times as great as the amount of diaminopimelate in the cell wall obtained by cultivation at a recommended cultivation temperature (specifically, 30°C or 37°C), or a temperature that makes the thickness of the cell wall at least 106% as thick as the thickness of the cell wall obtained by cultivation at a recommended cultivation temperature (specifically, 30°C or 37°C). The amount of diaminopimelate in the cell wall is obtained by quantifying diaminopimelate, which is amino acid that constitutes peptide and cross-links sugar chains in the cell wall, using a method known by a person skilled in the art. The thickness of the cell wall can be determined by an observation of lactic acid bacteria using an electronic microscope. It can be calculated using a formula, such as "(full length (minor axis) of the lactic acid bacterium - length of cytoplasm (length between the outer periphery of an inner membrane on one end of the minor axis and the outer periphery of an inner membrane on the opposite end of the minor axis))/2." A more preferable cultivation temperature is "a cultivation temperature that increases the amount of diaminopimelate in the cell wall to at least 1.5 times in comparison to the amount obtained by cultivation at the recommended cultivation temperature or that increases the thickness of the cell wall to at least 110% in comparison to the thickness obtained by cultivation at the recommended cultivation temperature", and the most preferable cultivation temperature is "a cultivation temperature that increases the amount of diaminopimelate in the cell wall to at least 1.65 times the amount obtained by cultivation at the recommended cultivation temperature or that increases the thickness of the cell wall to at least 115% in comparison to the thickness obtained by cultivation at the recommended cultivation temperature."

Any medium can be used for cultivation as long as lactic acid bacteria can grow in it. Mediums, such as milk, an MRS medium, a BL medium, a broth medium or a synthetic medium, can be used.

### Osmotic Pressure

In the method of the present invention for producing lactic acid bacteria having enhanced immunoregulating activities, the osmotic pressure of the medium is in the range of 500 to 1000 mOsm. Specifically, cultivation under an osmotic pressure of 500 to 1000 mOsm and at a desired cultivation temperature enhances immunoregulating activities.

Osmotic pressure can be upregulated by any method including the addition of ingredients, such as sorbitol, to the medium.

### Immunoregulating Activities

The lactic acid bacteria obtained by the present invention have significantly potent immunoregulating activities. When an object is described as having potent immunoregulating activities, it means that the object has, but is not limited to, at least one of (i) immunostimulating activity, (ii) immunosuppressing activity, (iii) immunobalanace optimization activity, and (iv) gut immunity stimulating activity, or preferably all of the above activities. These activities are not completely independent and they may correlate, as a person skilled in the art would readily understand.
(i) An immunostimulating activity is an activity of stimulating the immunological function when it is in a stationary or a deteriorated state. Examples of a deteriorated state of an immunological function include situations where the immunological function deteriorates due to old age, stress, fatigue, sleeplessness and other factors. The immunostimulating activity of the lactic acid bacteria of the present invention can be verified by the following: an activation of an animal's macrophage as the lactic acid bacteria of the present invention is added; an enhancement in the NK activity of the peripheral blood mononuclear cell (PBMC) in a human who is given bacteria for a specific period of time; and an enhanced production of IFN-α exhibiting an anti-virus activity. A specific method to assess an immunostimulating activity by macrophage activation is to measure interleukin-12 (IL-12) production. This method requires at least four to five specimens of lactic acid bacteria as samples. Specifically, two to three lactic acid bacteria samples of different levels are selected from five levels of bacteria samples classified by their final concentrations, which are 0.75, 2.25, 7.5, 22.5 or 75×10⁶ cells/mL, to be added to the macrophage. After 24 hours of cultivation, the IL-12 concentration of the supernatant of the culture is measured. The results for the level showing the highest value among the two to three samples that have strongly induced IL-12 production are selected as the subject of assessment.
(ii) An immunosuppressing activity is an activity of suppressing the excessive stimulation of immunological functions at its occurrence (specifically, allergy, atopy) to bring about a suitable immunological state. Examples include an activity of suppressing allergic reactions caused by antigens such as pollen and ticks.
(iii) An immunobalance optimization activity is an activity of optimizing the balance between cellular immunity and hormonal immunity. Examples include an activity of promoting or suppressing cytokine production, an activity of stimulating lymphocytes, an activity of enhancing a NK (natural killer) activity, an activity of improving the Th1/Th2 balance.
(iv) Gut immunity is a protection system on the intestinal mucosa for eliminating pathogenic factors that invade the intestine through the nose or the throat or by joining with objects such as food or drink. Specific examples include an activity of enhancing IgA production in the mucus, and a cytokine production-enhancing activity of immunocytes in the Peyer patch and the mesenteric lymph nodes.

### <Lactic Acid Bacteria>

The production method of the present invention provides lactic acid bacteria having enhanced immunoregulating activities.

The lactic acid bacteria obtained by the cultivating method of the present invention may be further processed as necessary. Other usable lactic acid bacteria having immunoregulating activities include lactic acid bacteria collected by centrifuging or filtering the medium after cultivation has ended (viable bacteria), lactic acid bacteria obtained by lyophilizing such bacteria, or lactic acid bacteria obtained by heating such bacteria.

A preferable administration method of lactic acid bacteria is oral administration. An exemplary dosage for a human may consist of 10 million to 1 trillion bacterial cells per a single administration, and more preferably, 100 million to 100 billion bacterial cells per a single administration. Administration may be once per day or divided into multiple times per day. The timing of intake is not particularly limited.

### <Foods or Drinks, Medicines or Cosmetics Containing Lactic Acid Bacteria>

The lactic acid bacteria produced by the method according to the present invention may be used as foods or
drinks, medicines, cosmetics or other products. A preferable implementation of th when they are used as foods or drinks, is health foods having immunoregulating activities. Lactic acid bacteria in foods or drinks can be mixed with various components known in the art, such as bases, adjuvants; sweeteners, acidulants, and Vitamins, to form a product that is adapted to the palate of the person consuming the bacteria. The bacteria can be provided in forms including a tablet, a capsule, a pill, powders, granules, a candy, a drop, a troche, gum, powder juice, health drink, a flavoring material, processed food, desserts or sweets. Embodiments of bacteria as foods or drinks allow daily intake of a composition containing the lactic acid bacteria produced by the method of the present invention to induce the immunoregulating functions of the composition and provide sustained health.

Medicines formulated from bacteria include immunostimulating agents and antiallergic agents. Further, medicines can be formulated by adding adjuvants known and commonly used in the art of pharmaceutical formulation to the base. Such adjuvants include an excipient, a binder, a disintegrator, a lubricant, a corrective, a solubilizer, a suspending agent, and a coating agent. Usable dosage forms include but are not limited to a pill, a tablet, a capsule, granules, powders, syrup, a suppository, an injection. Routes of administering the medicine include but are not limited to oral administration, rectal administration, and enteral administration.

Foods or drinks or medicines containing the lactic acid bacteria produced by the method of the present invention contain 1000 to 1 trillion, more preferably 100 million to 100 billion bacterial cells
per a single administration, but there is no actual upper limit to this number because the lactic acid bacteria of the present invention is extremely safe.

The present invention is described in detail but not limited by the Examples below.

### [Example 1]

### (Variations in Lactic Acid Bacteria Concentration, Amount of Cell Wall Components, and Interleukin-12 Inducing Activity Due to Differences in Cultivation Temperature)

### Lactic Acid Bacteria Cultivation and Bacterial Cell Preparation

*Lactobacillus pentosus* S-PT84 (FERM BP-10028) and *Lactobacillus pentosus* JCM1558^{T} were independently cultivated in MRS mediums at specified temperatures of 25°C to 43°C for 24 hours.

After the bacteria were cultivated, they were collected by centrifuging at 8000 rpm for 10 minutes, then washed with physiological saline and re-centrifuged. Subsequently, the bacteria were washed once with physiological saline and once with ion exchanged water. Then, a lactic acid bacteria suspension was prepared by re-suspending the bacteria in an appropriate amount of ion exchanged water to measure the final bacterial concentration (number of bacterial cells/mL). The suspension was subsequently sterilized at 95°C for 1 minute and then lyophilized. The total weight of the lyophilized suspension was measured to obtain the number of bacteria per a unit of weight. It is noted that the recommended cultivation temperature of *Lactobacillus pentosus* S-PT84 (FERM BP-10028) and *Lactobacillus pentosus* JCM1558^{T} is 30°C.

### Diaminopimelate Measurement Method

To the lyophilized bacterial cells, 6N of HCl was added. Hydrolysis was performed at 100°C for 20 hours. Then, the resulting product was evaporated to dryness using the centrifugal thickener (produced by Thermo SCIENTIFIC Co.). To the dried product, 0.05 N of HCl was added to obtain 1 mg dry cells/mL. The resulting product was filtered by a disc filter for pre-HPLC treatment with a pore size of 0.2 µm, and then subjected to the L-8800 Hitachi High Speed Amino Acid Analyzer. The index used for cell wall components is diaminopimelate (DAP), which is amino acid that constitutes peptide and cross-links the sugar chains of the cell wall.

### Interleukin-12 (IL-12) Inducing Activity Measurement

To a BALB/c mouse (8 weeks old, female), 2 mL of 4.05% thioglycolate was intraperitoneally administered. Four days later, the intraperitoneal macrophage was collected using PBS and adjusted to 2×10⁶ cells/mL in an RPMI1640 medium containing 10% FBS and then seeded in 48 well plates in an amount of 0.5 mL per well. Bacterial cells in an amount of 7.5×10⁶ cells/mL were added to each well, wherein the cells added to the respective wells were obtained by cultivation at respective specified cultivation temperatures. After 24 hours of cultivation, the IL-12 concentrations in the conditioned mediums were measured. IL-12 (p70) was measured, because the active form of IL-12 is p70, wherein subunit p35 binds to subunit p40. The instrument used to measure IL-12 is OptEIA mouse IL-12 measurement kit (produced by BD Pharmingen Co.).

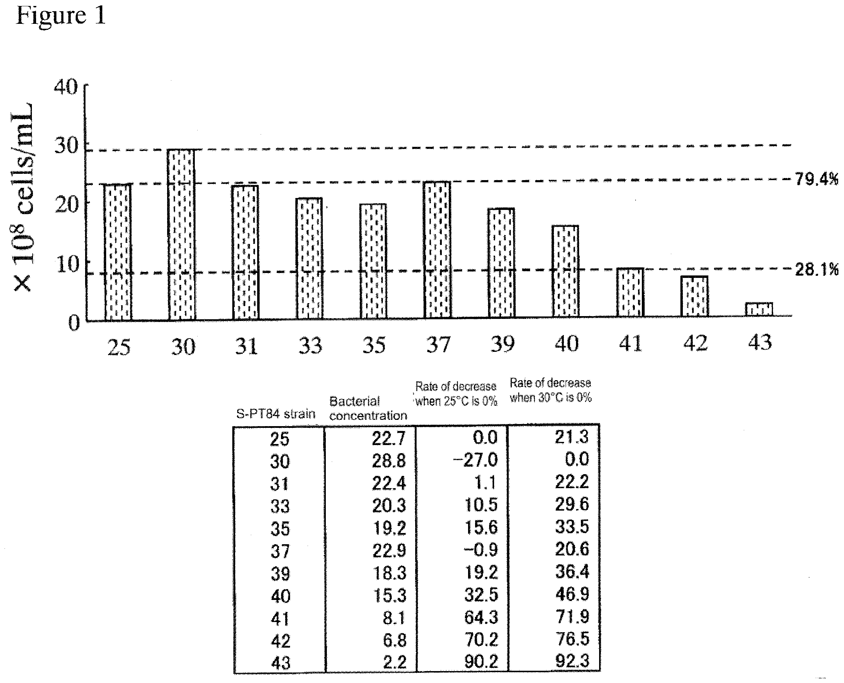

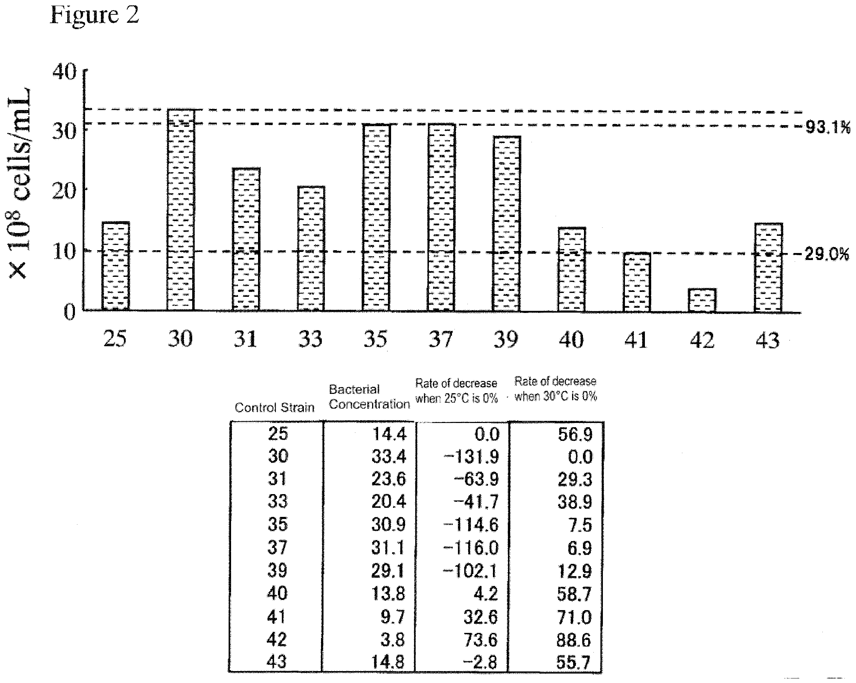

A result of the bacterial concentration of *Lactobacillus pentosus* S-PT84 (FERM BP-10028) after cultivation has ended is shown in Fig. 1. A result of the bacterial concentration of *Lactobacillus pentosus* JCM1558^{T} is shown in Fig. 2. Both S-PT84 (FERM BP-10028) and JCM1558^{T} showed a decrease of at least 30% in the bacterial concentration at 42°C from the bacterial concentration at 25°C, and a decrease of at least 75% from the bacterial concentration at 30°C.

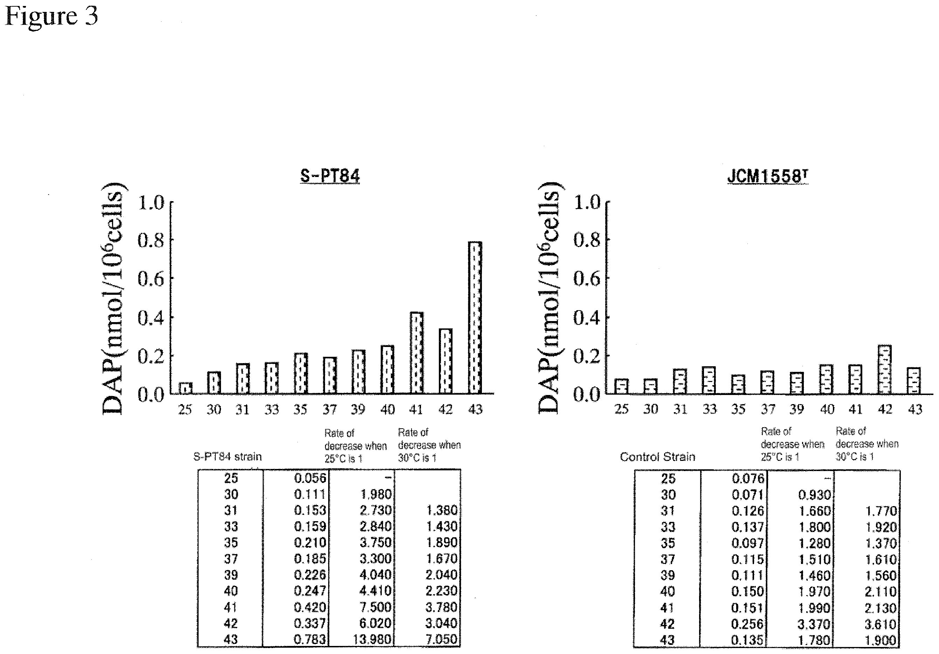

The measured result of DAP, which is an amino acid constituting the cell wall, is shown in Fig. 3. The DAP concentration in S-PT84 (FERM BP-10028) cultivated at 30°C increased to approximately twice that cultivated at 25°C. Although there was a small fluctuation, the concentration increased as the cultivation temperature increased. JCM1558^{T} cultivated at 31°C or higher showed an increase in the DAP concentration compared to the concentration in JCM1558^{T} cultivated at 25°C, and a cultivation at 42°C gave the maximum concentration. Further, the DAP concentration of cultivation at 42°C was 3.04 times as high as that of cultivation at 30°C for S-PT84 (FERM BP-10028) and 3.61 times as high for JCM1558^{T}.

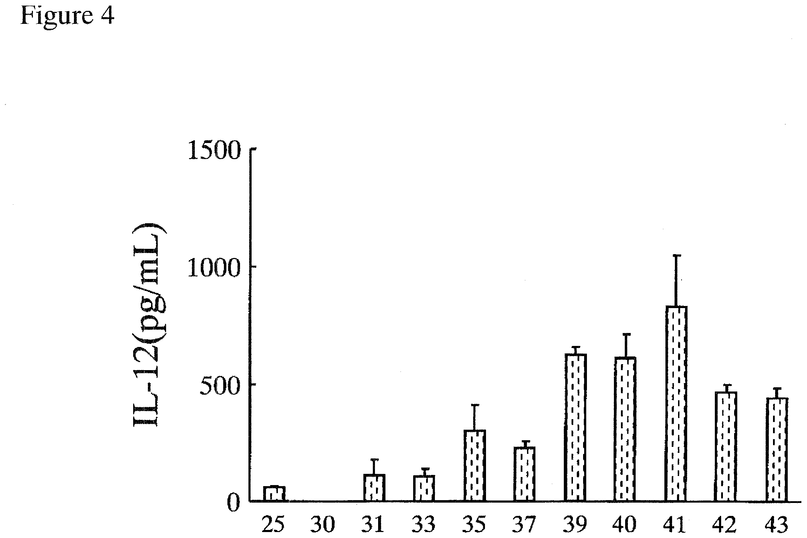

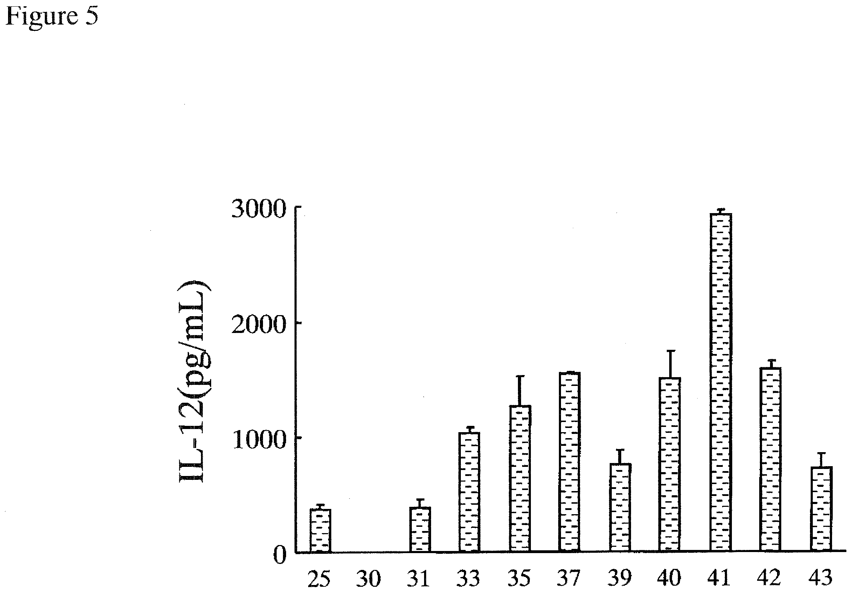

The immunostimulating activity was evaluated *in vitro* using IL-12 inducibility as an index. The results are shown in Figs. 4 and 5. For S-PT84 (FERM BP-10028), an increase in the DAP amount according to the increase of the cultivation temperature occurred at 30°C or higher. In addition, an increase of IL-12 inducibility in correlation with the DAP amount was observed until the temperature reached 41°C. JCM1558^{T} was similar to S-PT84 (FERM BP-10028) in that an increase in the DAP amount according to the increase of cultivation temperature occurred at 31°C or higher, and an increase of IL-12 induction was seen until the temperature reached 41°C. Both bacterial cells had higher IL-12 induction activities at 42°C and 43°C than at 25°C, but the activities at 42°C and 43°C were lower than those at 40°C. If activity is the only concern, cultivation at 31-43°C provides a sufficiently high immunostimulating activity, but if bacterial concentration is considered, a preferable temperature range to ensure a sufficient number of bacterial cells and to prepare a bacterial cell with a high immunostimulating activity is 31-41°C.

### [Example 2]

### (Variation in Lactic Acid Bacteria Concentration and Interleukin-12 Inducing Activity Due to Differences in Cultivation Temperature)

### Lactic Acid Bacteria Cultivation and Bacterial Cell Preparation

*Lactobacillus plantarum* JCM1149^{T} (Reference Example) was cultivated in MRS mediums at specified
temperatures of 30°C to 38°C and *Lactobacillus casei* JCM1134^{T} was cultivated in MRS mediums at specified temperatures of 25°C to 44°C, for 24 hours.

After the bacteria were cultivated, they were collected by centrifuging at 8000 rpm for 10 minutes, then washed with physiological saline and re-centrifuged. Subsequently, the bacteria were washed once with physiological saline and once with ion exchanged water. Then, a lactic acid bacteria suspension was prepared by re-suspending the bacteria in an appropriate amount of ion exchanged water to measure the final bacterial concentration (number of bacterial cells/mL). The suspension was subsequently sterilized at 95°C for 1 minute and then lyophilized. The total weight of the lyophilized suspension was then measured to obtain the number of bacteria per a unit of weight.

It is noted that the recommended cultivation temperature of *Lactobacillus plantarum* JCM1149^{T} (Reference Example) is 30°C and that *of Lactobacillus casei* JCM1134^{T} is 37°C.

### Interleukin-12 (IL-12) Inducing Activity Measurement

To a BALB/c mouse (8 weeks old, female), 2 mL of 4.05% thioglycolate was intraperitoneally administered. Four days later, the intraperitoneal macrophage was collected using PBS and adjusted to 2×10⁶ cells/mL in an RPMI1640 medium containing 10% of FBS and then seeded in 48 well plates in an amount of 0.5 mL per well. Bacterial cells in an amount of 7.5×10⁶ cells/mL were added to each well, wherein the cells added to the respective wells were obtained by cultivation at respective specified cultivation temperature. After 24 hours of cultivation, the IL-12 concentrations in the conditioned mediums were measured. IL-12 (p70) was measured, because the active form of IL-12 is p70, wherein subunit p35 binds to subunit p40. The instrument used to measure IL-12 is OptEIA mouse IL-12 measurement kit (produced by BD Pharmingen Co.).

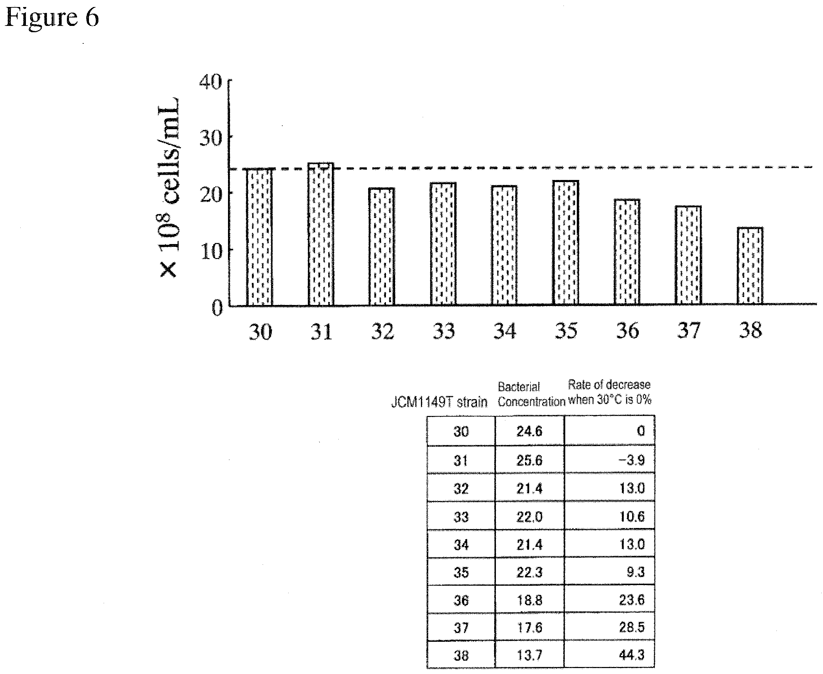

A result of the bacterial concentration after cultivation has ended of *Lactobacillus plantarum* JCM1149^{T} is shown in Fig. 6 (Reference Example). The bacterial concentration at 38°C decreased by 44.3% from that at 30°C.

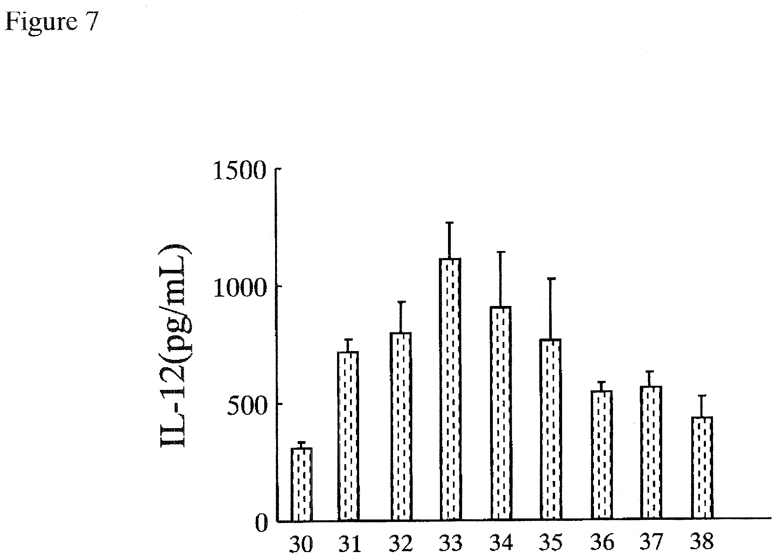

The immunolostimulating activity was evaluated *in vitro* using IL-12 inducibility as an index. The result for *Lactobacillus plantarum* JCM1149^{T} is shown in Fig. 7 (Reference Example) The IL-12 inducibility showed high values at 31°C to 38°C compared to the value at 30°C, and its peak was at 33 °C. In view of both the bacterial concentration and the immunostimulating activity, a preferable temperature range to ensure a sufficient number of bacterial cells and to prepare a bacterial cell with a high immunostimulating activity was considered to be 31-38°C.

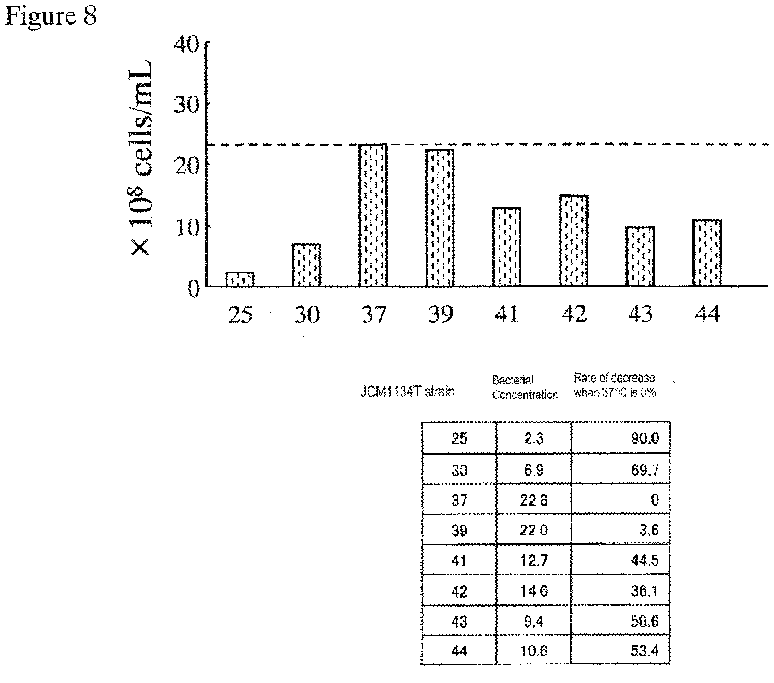

A result of the bacterial concentration after cultivation has ended of *Lactobacillus casei* JCM1134^{T} is shown in Fig. 8. The bacterial concentration at 44°C decreased by 53.4% from that at 37°C.

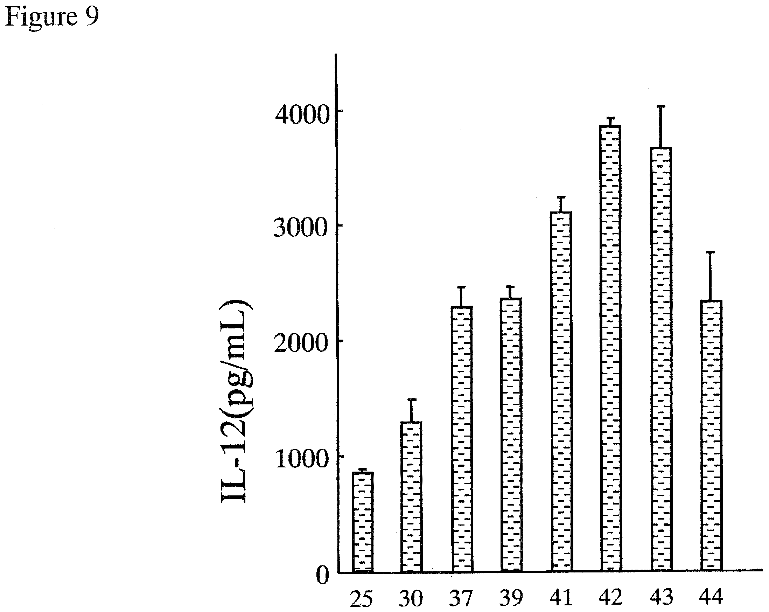

The immunolostimulating activity was evaluated *in vitro* using IL-12 inducibility as the index. The result for *Lactobacillus casei* JCM1134^{T} is shown in Fig. 9. The IL-12 inducibility showed high values at 39°C to 44°C compared to that at 37°C, and its peak was at 42°C. In view of both the bacterial concentration and the immunostimulating activity, a preferable temperature range to ensure a sufficient number of bacterial cells and to prepare a bacterial cell with a high immunostimulating activity was considered to be 41-44°C.

### [Example 3]

### (Variations in Cell Wall Synthetic Enzyme Gene Expression Analysis and Cell Wall Component Amount Due to Differences in Cultivation Temperature)

### Primer for Analyzing Enzyme Expression Related to Cell Wall Synthesis

Q-RT-PCR primers were prepared for phospho-N-acetylmuramoyl-pentapeptide transferase (mraY), penicillin binding protein 1A (pbp1A), and penicillin binding protein 2A (pbp2A) based on the gene information of *Lactobacillus,* which has a publicly disclosed genome information. The primers used are mraY116:aggaaggtcctaagtggca/ mraY895:actcgctccaacccttcat for mraY, pbp1A304:gccgtcgtctcaatcgaaga/ pbp1A1724:gtaccagtcttaccagcttg for pbp1A pbp2A592:gcgatgtatttgaataacgc/ pbp2A1688:agcatcatactggtcatttc for pbp2A. Further, all expression analysis used 16S rRNA gene expression as the control, and primers were designed based on the 16S rRNA gene information of *Lactobacillus pentosus* S-PT84 (FERM BP-10028) (S-PT84-16S-f:accgacttcgggtgttacaa/S-PT84-16S- r:cgcctacatgaagtcggaat).

### Conditions for Inducing Expression

Temperature difference: Lactic acid bacteria were cultivated in an MRS medium at 30°C for 16 hours. Subsequently, MRS mediums, each in an amount of 100 mL, were inoculated with 2 mL of the culture, and then were cultivated at 30°C and 37°C for 4 hours.

### RNA Extraction and Q-RT-PCR Condition

RNA extraction was conducted by using RNeasy™ Mini Kit (produced by QIAGEN Co.) in accordance with the kit's protocol. To eliminate contamination of DNA into the obtained RNA extract, RNase-Free DNase Set (produced by QIAGEN Co.) were added to the extract and the mixture was incubated at room temperature for 15 minutes to decompose the DNA contaminated therein. The obtained DNase-processed RNA solution was used as the template. The template used for 16S rRNA was a DNase-processed RNA solution that was diluted to a hundredth of the original concentration. 2 µl of a template was added to 18 µl of a reaction solution containing 10 µM of each primer to perform Q-RT-PCR. The Q-RT-PCR was performed by using One Step SYBR™ Prime Script™ RT-PCR Kit II (produced by TaKaRa Co.) in accordance with the kit's instruction. Applied Biosystems 7300/7500 Real-Time PCR System was used as the Q-RT-PCR device.

### Cell Wall Component Amount

Temperature: Lactic acid bacteria were cultivated in an MRS medium at 30°C for 16 hours. Subsequently, MRS mediums, each in an amount of 100 mL, were inoculated with 2 mL of culture solution, and then were cultivated at 30°C and 37°C for 4 hours.

Osmotic Pressure: Lactic acid bacteria were cultivated in an MRS medium at 30°C for 16 hours. Subsequently, MRS mediums, each in an amount of 100 mL containing either 1, 3, 5 or 10% solbitol (the osmotic pressure is respectively 490, 614, 742, 2079 mOsm) (the control is a plain MRS medium) and inoculated with 2 mL of a culture solution of 2 mL, were cultivated at 30°C and 37°C for 24 hours. OD660 was also measured.

Washing and Lyophilization: Bacteria were collected by centrifuging at 8000 rpm for 10 minutes, then washed with physiological saline and re-centrifuged. Subsequently, the bacteria were washed once with physiological saline and once with ion exchanged water. Then, a lactic acid bacteria suspension was prepared by re-suspending the bacteria in an appropriate amount of ion exchanged water to measure the final bacterial concentration (number of bacterial cells/mL) using a hemacytometer. Then, the lactic acid bacteria solutions were frozen at -80°C for 16 hours, and lyophilized for 3 days. The lyophilized bacteria cells were ground in a mortar to be used as samples for amino acid analyses.

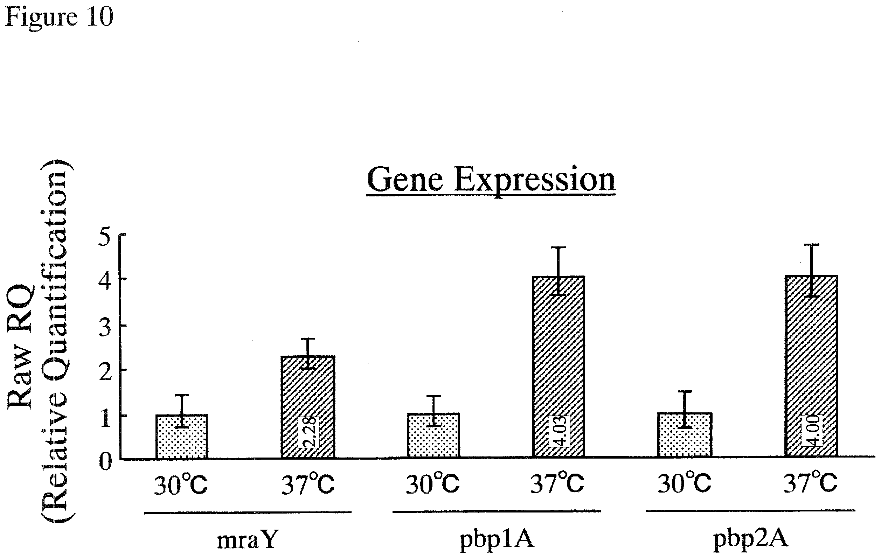

As a result of relative quantification performed in a temperature change experiment using 30°C as the control, an increase in the expression amount was observed at 37°C. The increase was by 2.28 times for mraY, 4.03 times for pbp1A, and 4.00 times for pbp2A (Fig. 10).

Diaminopimelate, which is an index for the amount of cell wall components, will be indicated as DAP herein.

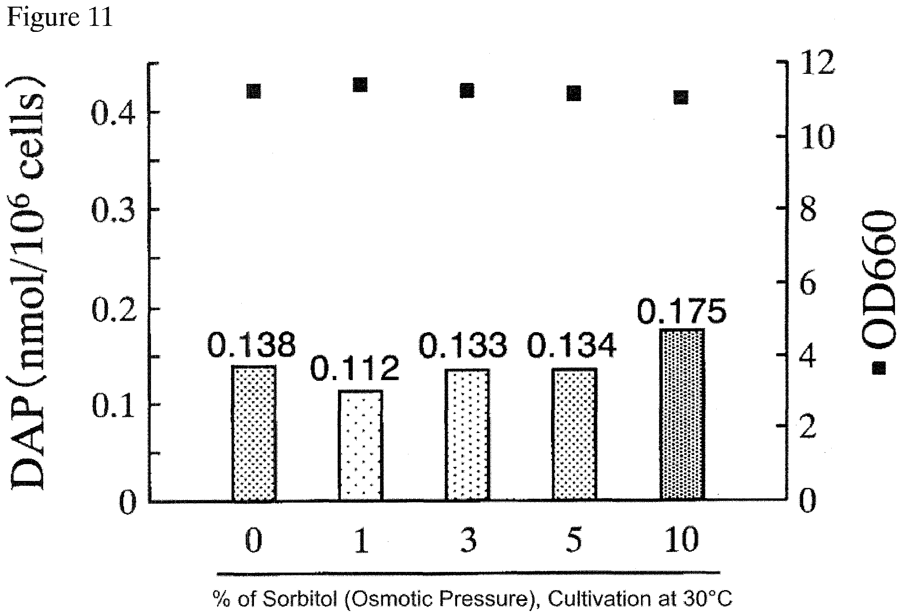

In a cultivation at 30°C, the control (solbitol-free MRS medium, 24 hours) produced 0.138 nmol DAP/10⁶ cells (OD660: 11.2), whereas the cultivation using MRS medium that contains only 10% solbitol produced 0.175 nmol DAP/10⁶ cells (OD660: 11.0). In other words, a DAP increase by 1.27 times was observed (Fig. 11).

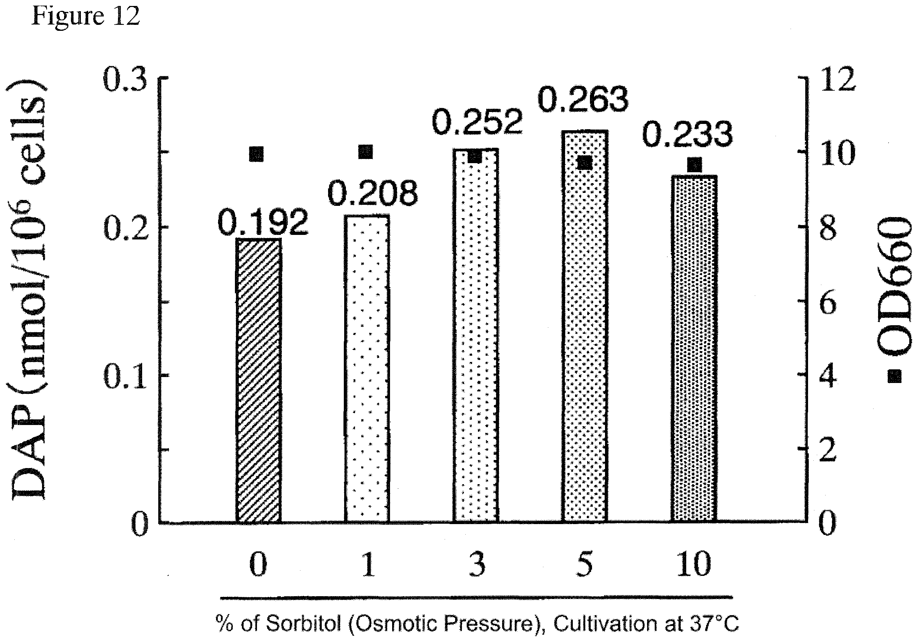

Meanwhile, a cultivation at 37°C produced 0.192 nmol DAP/10⁶ cells for the control (solbitol-free MRS medium, 24 hours), whereas the cultivation with solbitol contents of 1%, 3%, 5%, and 10% respectively produced 0.208 nmol DAP/10⁶ cells, 0.252 nmol DAP/10⁶ cells, 0.263 nmol DAP/10⁶ cells, 0.233 nmol DAP/10⁶ cells. In other words, respective DAP increases by 1.08 times, 1.31 times, 1.37 times and 1.21 times were observed (Fig. 12).

### [Example 4]

(Measurement of Variations in Cell Wall Thickness by Differences in Cultivation Temperature)

### Lactic Acid Bacteria Cultivation and Fixation

S-PT84 (FERM BP-10028) was cultivated in MRS mediums at 25, 30 and 37°C for 24 hours. JCM1558^{T} was cultivated in MRS mediums at 25 and 37°C for 24 hours. After cultivation has ended, bacteria were collected by centrifuging at 8000 rpm for 10 minutes, then washed with physiological saline and re-centrifuged. Subsequently, the bacteria were washed once with physiological saline and once with ion exchanged water. Then, PBS containing 2% glutaraldehyde/2% paraformaldehyde was added to the pellet to fix the bacteria.

### Electron Micrograph

After the pellet was produced by centrifuging, and washed with distilled water, postfixation was performed using a 1% potassium permanganate aqueous solution (4°C, 1 hour). The pellet was rewashed with distilled water and dehydrated by using acetone. Then, it was thermally polymerized with Quetol 651 (epoxy resin) (60°C, 24 hours). Ultrathin sections were cut using an ultramicrotome and double stained by a stain of uranyl acetate/lead. TEM images of the sections were taken using JOEL JEM1200EX.

### Cell Wall Thickness Measurement

The cell wall of a lactic acid bacterium was defined as follows. "The longest minor axis of a lactic acid bacterium is selected. The region between the outer periphery of the inner membrane and the visible extracellular limit on that axis is defined as the cell wall". Based on this definition, the cell wall thickness was calculated by the following formula. Cell wall thickness: (Full length (minor axis) of the lactic acid bacterium - length of cytoplasm (length between the outer periphery of an inner membrane on one end of the minor axis and the outer periphery of an inner membrane on the opposite end of the minor axis))/2.

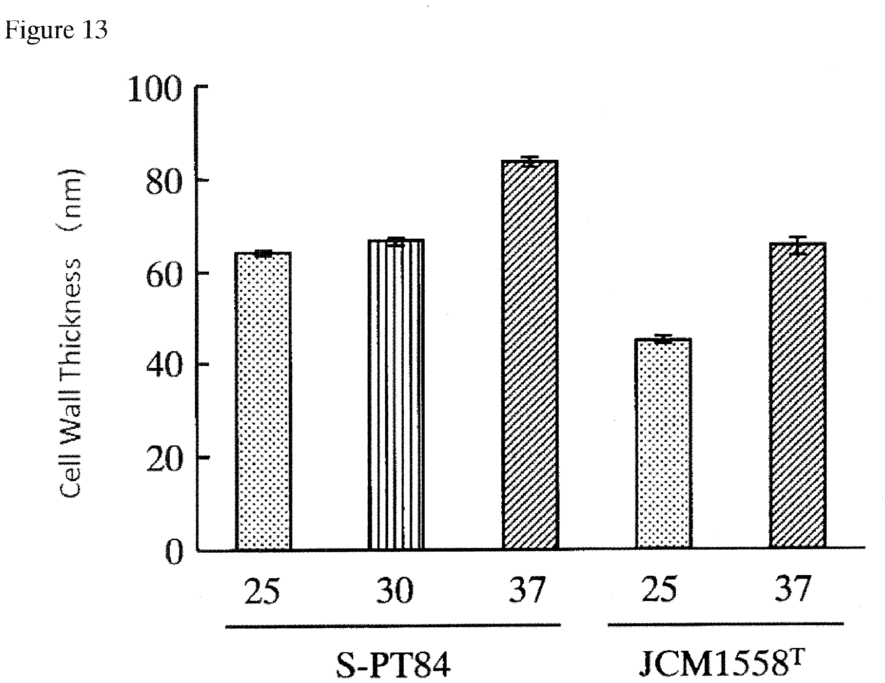

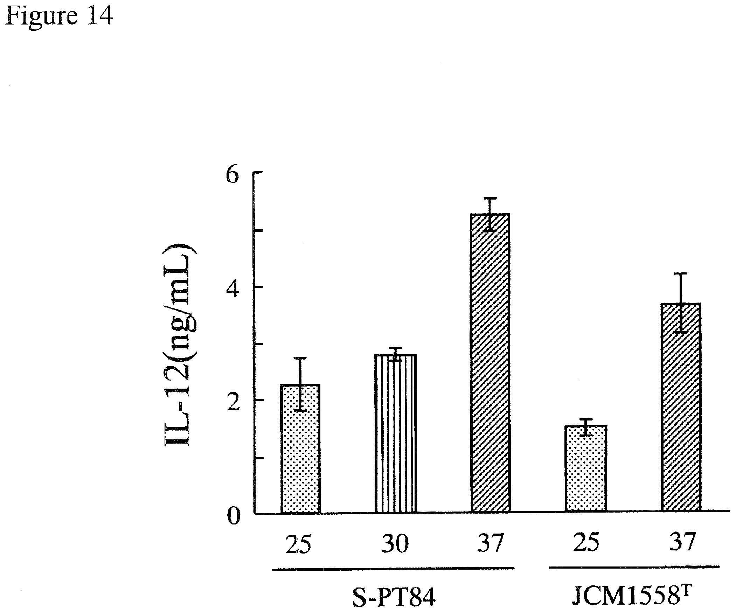

The cell wall thicknesses of S-PT84 (FERM BP-10028) at temperatures of 25, 30, 37°C were respectively 64.1, 66.4 and 83.5 nm, and those of JCM1558^{T} at temperatures of 25°C, 37°C were respectively 45.2 and 65.4nm. Cell wall thickness in both cases increased in a temperature-dependent manner (Fig. 13). Further, an evaluation of IL-12 inducibility using the above method gave results of 2.3, 2.8, 5.2 ng/mL respectively for cultivations of S-PT84 (FERM BP-10028) at 25, 30, 37°C, and 1.5, 3.7 ng/mL respectively for cultivations of JCM1558^{T} at 25°C, 37°C. The results show that the immunostimulating activity increases in association with the increase of the cell wall thickness (Fig. 14).

### [Example 5]

### (Evaluation at Actual Production Scale)

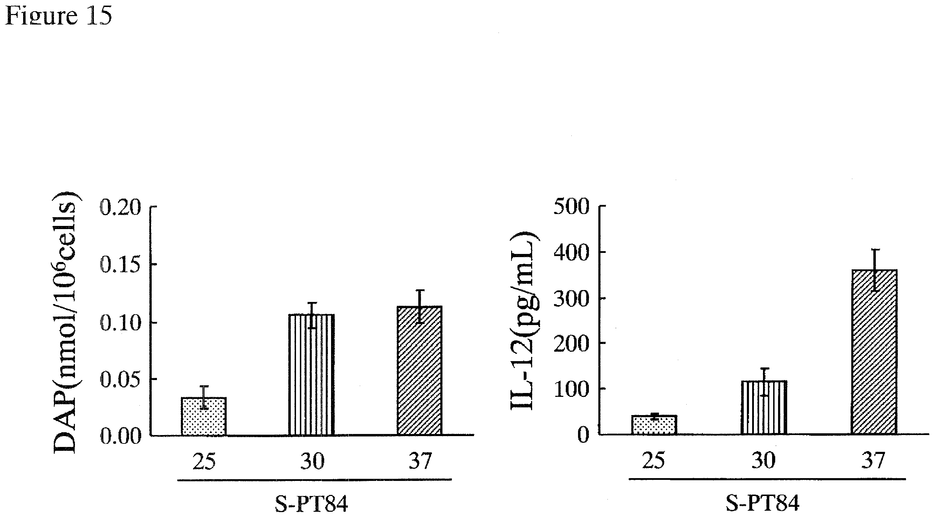

An MRS medium was used in Example 1. In addition to such Example, cultivation at an actual production scale (1000 L cultivation) was performed to evaluate whether the same phenomenon would occur. S-PT84 (FERM BP-10028) was cultivated in mediums comprising mixtures of glucose, yeast extract (AROMILD™, SK yeast extract Hi-K) and ion exchanged water at respective temperatures of 25°C (24-26°C), 30°C (29-31°C) and 37°C (36-38°C) for 24 hours (20-28 hours). Three lots were created for each cultivation temperature to evaluate the cell wall component amount and IL-12 inducibility. The result showed that the cell wall amounts were respectively 0.033, 0.106 and 0.112 nmol DAP/10⁶ cells for temperatures of 25, 30 and 37°C, and that the cell wall amounts increased in a cultivation temperature-dependent manner. Further, the IL-12 inducibility values were respectively 38.4, 98.4 and 359.0 pg/mL, and the inducibility increased relative in a cultivation temperature-dependent manner (Fig. 15). This result showed that the same phenomenon would occur even if the medium differs.

### [Example 6]

### (Immunostimulating Effects on Human)

### Test Method

Immunostimulating effects on a human were evaluated using S-PT84 (FERM BP-10028) produced at a cultivation temperature (37°C) that increases the amount of cell wall components and the cell wall thickness, as demonstrated by the previous Examples.

S-PT84 (FERM BP-10028) that was diluted by dextrin to a concentration of one tenth the original was used as the starting material to produce tablets containing either 500 million bacterial cells, 1.5 billion bacterial cells or 4.5 billion bacterial cells by adding excipients as necessary. Subjects having peripheral blood PBMCs with NK activity below 30% were selected by a prior screening and divided into groups with approximately the same average NK activity value. Blood was collected at the following timings: before the intake of lactic acid bacteria; two weeks after bacteria intake has started; and four weeks after bacteria intake has started. PBMC was isolated from the collected blood and its immunological functions (NK activity, IFN-α production capacity 24 hours after HVJ stimulation, and ratio of IFN-γ/IL-4 production 24 hours after PHA stimulation) were assessed.

### NK Activity

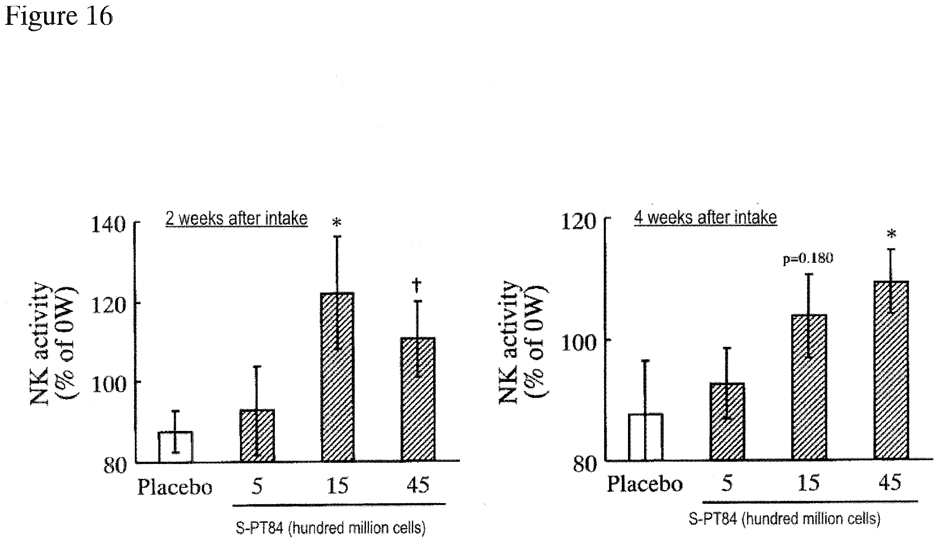

A significant NK activity enhancement in comparison to that before bacteria intake resulting from the intake of 1.5 billion S-PT84 (FERM BP-10028) cells was observed 2 weeks after bacteria intake has started. An increase in the activity depending on the S-PT84 (FERM BP-10028) amount was observed 4 weeks after bacteria intake has started, and an intake of 4.5 billion bacteria cells produced a significant NK activity enhancement in comparison to that before bacteria intake has started (Fig. 16).

### IFN-α Production Capacity

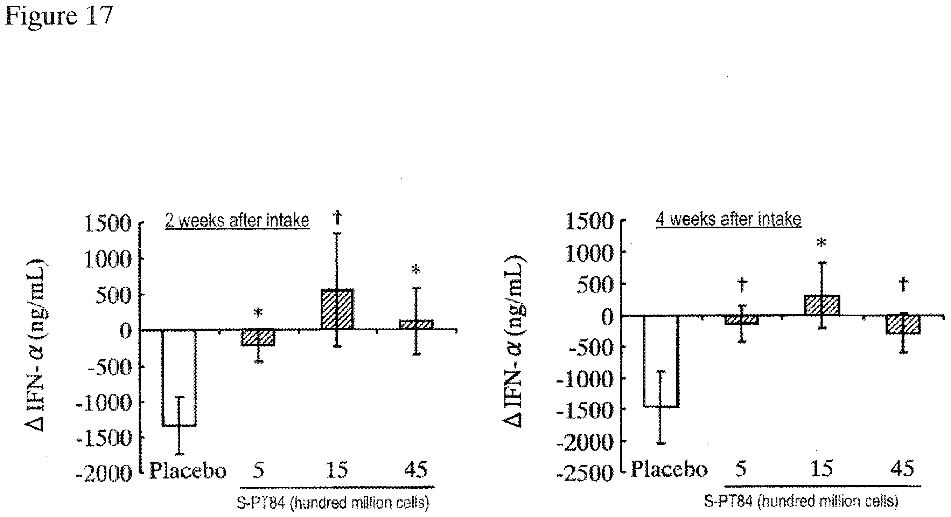

The group taking placebo experienced decrease in their IFN-α production capacity at 2 or 4 weeks after the start of the test, but all groups taking S-PT84 (FERM BP-10028) exhibited a maintained or enhanced level of IFN-α production capacity. Specifically, an increase in IFN-α generation was evident in the group that took 1.5 billion cells (Fig. 17).

### IFN-γ, IL-4 Production Capacity

Helper T cells in the living body include type 1 helper T cells (Th1) and type 2 helper T cells (Th2). Th1 cells and Th2 cells are known to produce IFN-γ and IL-4 respectively, so the balance of Th1/Th2 cells was calculated from the ratio of IFN-γ/IL-4.

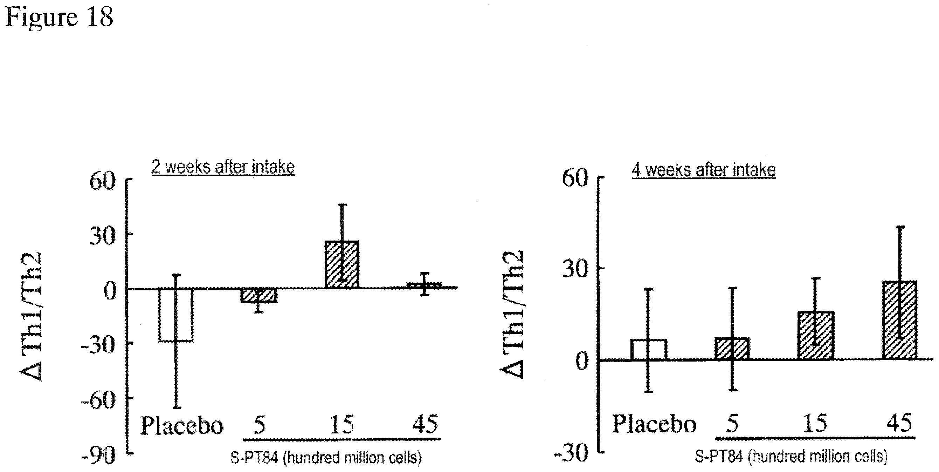

In the 2nd week, the group taking placebo tended towards Th2, and the group that took 1.5 billion cells of S-PT84 (FERM BP-10028) shifted to the Th1 side. The result of the 4th week showed a tendency towards Th1 which is dependent on the S-PT84 (FERM BP-10028) amount (Fig. 18).

### [Example 7]

### (Assessment of Other Types of Lactic Acid Bacteria)

Examples 1, 3 to 5 were performed using 2 strains of *Lactobacillus pentosus.* In addition to such Examples, other genera were evaluated whether the same phenomenon would occur. *Lactobacillus plantarum* JCM1149^{T} (Reference Example), *Lactobacillus brevis* JCM1059^{T}, *Lactobacillus fermentum* IFO3656, *Lactobacillus casei* JCM1134^{T} were each cultivated in respective MRS mediums at specified temperatures (at 25, 30 or 37°C for lactic acid bacteria whose recommended cultivation temperature is 30°C, and at 30°C or 42°C for lactic acid bacteria whose recommended cultivation temperature is 37°C) for 24 hours. TEM photographs of these bacterial cells were taken by the above mentioned process using an electronic microscope to measure the cell wall thickness as well as evaluate the IL-12 inducing activity.

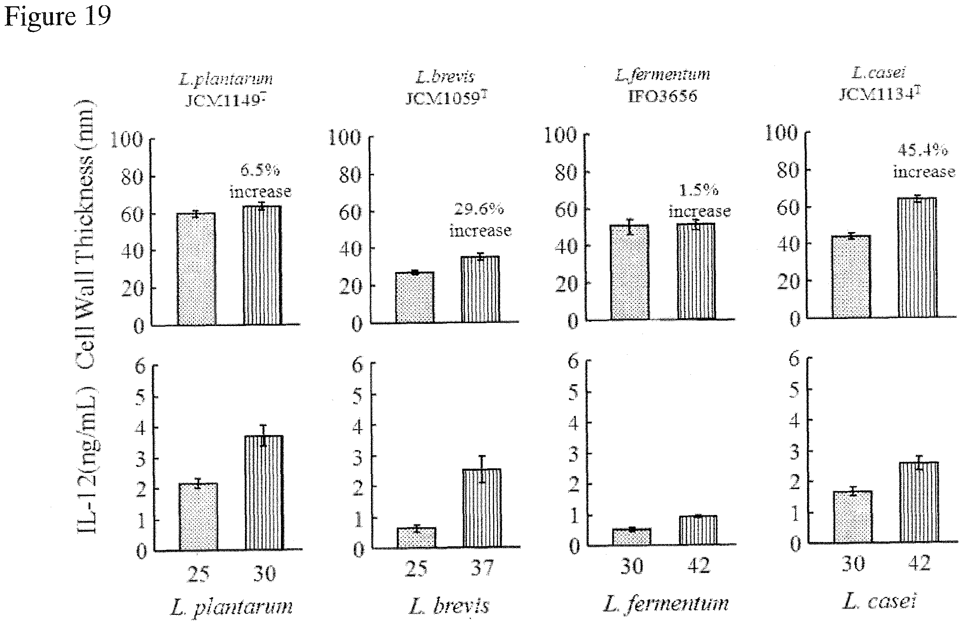

*Lactobacillus plantarum* JCM1149^{T} (Reference Example) and *Lactobacillus brevis* JCM1059^{T} whose recommended cultivation temperature is 30°C exhibited increases in the cell wall thickness and IL-12 inducing activity as the cultivation temperature increased, so a phenomenon similar to that of *Lactobacillus peretosus* was observed (Fig. 19). Surprisingly, the exact same phenomenon was observed in *Lactobacillus fermentum* IFO3656 and *Lactobacillus casei* JCM1134^{T} whose recommended cultivation temperature is 37°C (Fig. 19). The above results showed that the increase in the cell wall thickness and the enhancement of immunoregulating activities due to an increase in cultivation temperature are phenomena occurring across genera.

### INDUSTRIAL APPLICABILITY

Cultivation at temperatures higher than the recommended cultivation temperature of the lactic acid bacteria to be used increases the cell wall amount and the cell wall thickness. Lactic acid bacteria with enhanced immunoregulating activities are obtained in correlation to these increases. The method of the present invention enhances the immunoregulating functions of lactic acid bacteria without requiring any special medium.
This enhancement allows lactic acid bacteria exhibiting excellent immunoregulating activities in a human, or products including such bacteria, such as foods or drinks, medicines or cosmetics to be provided.

## Claims

1. A method for producing lactic acid bacteria having enhanced immunoregulating activities comprising the step of:
cultivating lactic acid bacteria under an osmotic pressure of 500 to 1000 mOsm and at a cultivation temperature that is at least 1°C higher than a recommended cultivation temperature of the lactic acid bacteria and that gives a bacterial concentration 25 to 95% of a bacterial concentration obtained by cultivation at the recommended cultivation temperature, thereby lactic acid bacteria having enhanced immunoregulating activities are obtained,
wherein the lactic acid bacteria are bacteria of *Lactobacillus pentosus, Lactobacillus brevis, Lactobacillus fermentum* or *Lactobacillus casei*,
wherein for *Lactobacillus pentosus* or *Lactobacillus brevis* the recommended cultivation temperature is 30°C and the cultivation temperature is 31°C to 41°C,
and wherein for *Lactobacillus fermentum* or *Lactobacillus casei* the recommended cultivation temperature is 37°C and the cultivation temperature is 41 °C to 44°C.

2. A method for producing lactic acid bacteria having enhanced immunoregulating activities comprising the step of:
cultivating lactic acid bacteria under an osmotic pressure of 500 to 1000 mOsm and at a cultivation temperature that increases diaminopimelate in a cell wall to at least 1.35 times in comparison to an amount obtained by cultivation of the lactic acid bacteria at a recommended cultivation temperature, or at a cultivation temperature that increases a cell wall thickness to at least 106% in comparison to a cell wall thickness obtained by cultivation of the lactic acid bacteria at a recommended cultivation temperature, thereby lactic acid bacteria having enhanced immunoregulating activities are obtained,
wherein the lactic acid bacteria are bacteria of *Lactobacillus pentosus, Lactobacillus brevis, Lactobacillus fermentum* or *Lactobacillus casei*,
wherein for *Lactobacillus pentosus* or *Lactobacillus brevis* the recommended cultivation temperature is 30°C and the cultivation temperature is 31 °C to 41 °C,
and wherein for *Lactobacillus fermentum* or *Lactobacillus casei* the recommended cultivation temperature is 37°C and the cultivation temperature is 41°C to 44°C.

3. The method according to claim 1 or 2, wherein the lactic acid bacteria is *Lactobacillus pentosus* S-PT84 or *Lactobacillus pentosus* JCM1558^{T}.

## Patentansprüche

1. Ein Verfahren zur Herstellung von Milchsäurebakterien mit verstärkter immunregulierender Wirkung, umfassend den Schritt:
Kultivieren von Milchsäurebakterien unter einem osmotischen Druck von 500 bis 1000 mOsm und bei einer Kultivierungstemperatur, die mindestens 1°C höher ist als eine empfohlene Kultivierungstemperatur der Milchsäurebakterien und die eine bakterielle Konzentration von 25 bis 95% einer bakteriellen Konzentration, die durch Kultivieren bei der empfohlenen Kultivierungstemperatur erhalten wird, ergibt, wodurch Milchsäurebakterien mit verstärkter immunregulierender Wirkung erhalten werden,
wobei die Milchsäurebakterien Bakterien von *Lactobacillus pentosus, Lactobacillus brevis, Lactobacillus fermentum* oder *Lactobacillus casei* sind,
wobei für *Lactobacillus pentosus* oder *Lactobacillus brevis* die empfohlene Kultivierungstemperatur 30°C beträgt und die Kultivierungstemperatur 31°C bis 41°C beträgt,
und wobei für *Lactobacillus fermentum* oder *Lactobacillus casei* die empfohlene Kultivierungstemperatur 37°C beträgt und die Kultivierungstemperatur 41 °C bis 44°C beträgt.

2. Ein Verfahren zur Herstellung von Milchsäurebakterien mit verstärkter immunregulierender Wirkung, umfassend den Schritt:
Kultivieren von Milchsäurebakterien unter einem osmotischen Druck von 500 bis 1000 mOsm und bei einer Kultivierungstemperatur, die Diaminopimelat in einer Zellwand auf mindestens das 1,35-Fache verglichen mit einer durch Kultivierung der Milchsäurebakterien bei einer empfohlenen Kultivierungstemperatur erhaltenen Menge erhöht, oder bei einer Kultivierungstemperatur, die eine Zellwanddicke auf mindestens 106% verglichen mit einer Zellwanddicke, die durch Kultivieren der Milchsäurebakterien bei einer empfohlenen Kultivierungstemperatur erhalten wird, erhöht, wodurch Milchsäurebakterien mit verstärkter immunregulierender Wirkung erhalten werden,
wobei die Milchsäurebakterien Bakterien von *Lactobacillus pentosus, Lactobacillus brevis, Lactobacillus fermentum* oder *Lactobacillus casei* sind,
wobei für *Lactobacillus pentosus* oder *Lactobacillus brevis* die empfohlene Kultivierungstemperatur 30°C beträgt und die Kultivierungstemperatur 31°C bis 41°C beträgt,
und wobei für *Lactobacillus fermentum* oder *Lactobacillus casei* die empfohlene Kultivierungstemperatur 37°C beträgt und die Kultivierungstemperatur 41°C bis 44°C beträgt.

3. Das Verfahren nach Anspruch 1 oder 2, wobei die Milchsäurebakterien *Lactobacillus pentosus* S-PT84 oder *Lactobacillus pentosus* JCM1558^{T} sind.

## Revendications

1. Méthode pour la production de bactéries lactiques ayant des activités immunorégulatrices augmentées comprenant l'étape de :
culture des bactéries lactiques sous une pression osmotique de 500 à 1000 mOsm et à une température de culture qui est au moins 1 °C supérieure à une température de culture recommandée des bactéries lactiques et qui donne une concentration bactérienne de 25 à 95 % d'une concentration bactérienne obtenue par culture à la température de culture recommandée, des bactéries lactiques dotées d'activités immunorégulatrices augmentées étant ainsi obtenues,
dans laquelle les bactéries lactiques sont des bactéries de *Lactobacillus pentosus, Lactobacillus brevis, Lactobacillus fermentum* ou *Lactobacillus casei*,
dans laquelle pour *Lactobacillus pentosus* ou *Lactobacillus brevis* la température de culture recommandée est de 30 °C et la température de culture est de 31 °C à 41 °C,
et dans laquelle pour *Lactobacillus fermentum* ou *Lactobacillus casei* la température de culture recommandée est de 37 °C et la température de culture est de 41 °C à 44 °C.

2. Méthode pour la production de bactéries lactiques ayant des activités immunorégulatrices augmentées comprenant l'étape de :
culture des bactéries lactiques sous une pression osmotique de 500 à 1000 mOsm et à une température de culture qui augmente le diaminopimélate dans une paroi cellulaire d'au moins 1,35 fois en comparaison à une quantité obtenue par culture des bactéries lactiques à une température de culture recommandée, ou à une température de culture qui augmente l'épaisseur d'une paroi cellulaire d'au moins 106 % en comparaison à une épaisseur de paroi cellulaire obtenue par culture des bactéries lactiques à une température de culture recommandée, des bactéries lactiques dotées d'activités immunorégulatrices augmentées étant ainsi obtenues,
dans laquelle les bactéries lactiques sont des bactéries de *Lactobacillus pentosus, Lactobacillus brevis, Lactobacillus fermentum* ou *Lactobacillus casei*,
dans laquelle pour *Lactobacillus pentosus* ou *Lactobacillus brevis* la température de culture recommandée est de 30 °C et la température de culture est de 31 °C à 41 °C,
et dans laquelle pour *Lactobacillus fermentum* ou *Lactobacillus casei* la température de culture recommandée est de 37 °C et la température de culture est de 41 °C à 44 °C.

3. Méthode selon la revendication 1 ou 2, dans laquelle la bactérie lactique est *Lactobacillus pentosus* S-PT84 ou *Lactobacillus pentosus* JCM1558^{T}.
